# EUROPÄISCHE PATENTSCHRIFT

(11) **EP 3 180 308 B1**
(45) Veröffentlichungstag und Bekanntmachung des Hinweises auf die Patenterteilung: **18.04.2018**
(21) Anmeldenummer: 15745198.0
(22) Anmeldetag: 31.07.2015
(51) Int. Cl.: C07C 209/48, C07C 211/15

(54) **VERFAHREN ZUR HERSTELLUNG VON 2,2-DIFLUORETHYLAMIN**
METHOD FOR PRODUCING 2,2-DIFLUOROETHYLAMINE
PROCÉDÉ DE PRÉPARATION DE 2,2-DIFLUOROÉTHYLAMINE

(30) Priorität: 12.08.2014 EP 14180662
(43) Veröffentlichungstag der Anmeldung: 21.06.2017
(73) Patentinhaber: BASF SE, 67056 Ludwigshafen am Rhein (DE)
(72) Erfinder: REISSNER, Thomas, 68199 Mannheim (DE); ALTENHOFF, Ansgar Gereon, 69120 Heidelberg (DE); MUELLER, Christian, 68167 Mannheim (DE)
(74) Vertreter: BASF IP Association
(86) Internationale Anmeldenummer: PCT/EP2015/067675
(87) Internationale Veröffentlichungsnummer: WO 2016/023773

(56) Entgegenhaltungen:
- WO-A1-2011/069994

## Beschreibung

Die vorliegende Erfindung betrifft ein Verfahren zur Herstellung von 2,2-Difluorethylamin durch Hydrierung von Difluoracetonitril.

2,2-Difluorethylamin und seine Salze sind wichtige Zwischenprodukte zur Herstellung von Wirkstoffen, insbesondere von agrochemischen Wirkstoffen.

Zur Herstellung aliphatischer, primärer Amine sind verschiedene Methoden bekannt. Diese basieren auf dem Einsatz verschiedener Ausgangsstoffe, wie etwa Alkohole, Carbonylverbindungen, Nitrile oder Alkylhalogenide. Eine gängige Methode ist die Hydrierung von Nitrilen, da die entsprechenden Nitrile oftmals leichter zugänglich sind als etwa die entsprechenden Alkohole oder Carbonylverbindungen. Bei der Nitrilhydrierung werden üblicherweise Edelmetallkatalysatoren (Palladium, Platin, Rhodium), Nickel- oder Kobaltkatalysatoren, sowie auch Eisenkatalysatoren eingesetzt. Während Edelmetallkatalysatoren sehr milde Reaktionsbedingungen ermöglichen, erfordern Nickel- oder Kobaltkatalysatoren einen Druck von bis zu 25 MPa abs und eine Temperatur von bis zu 180°C. Eisenkatalysatoren erfordern teilweise noch höhere Drücke von 30 bis 35 MPa abs. Nickel, Kobalt und Eisen haben dafür den Vorteil, dass sie leichter verfügbar sind als Edelmetalle.

Bei der Nitrilhydrierung bildet sich intermediär zunächst das entsprechende Imin, welches dann weiter zum primären Amin hydriert wird. Durch Nebenreaktionen werden jedoch nicht nur die primären Amine sondern auch sekundäre und tertiäre Amine gebildet. Sekundäres Amin entsteht dabei nach einem allgemein anerkannten Mechanismus durch Addition des bereits gebildeten primären Amins an die Imin-Zwischenstufe unter Abspaltung von Ammoniak und nachfolgender Hydrierung. Tertiäres Amin entsteht in analoger Weise. Eine gute Zusammenfassung hierzu ist in Ullmann's Encyclopedia of Industrial Chemistry, 2012, Wiley-VCH Verlag GmbH & Co. KGaA, "Amines, Aliphatic", chapter 3 "General Production Methods" gegeben.

S. Gomez et al. in Adv. Synth. Catal. 2002, 344, No. 10, Seiten 1037 bis 1057 untersuchten den Mechanismus der Nitrilhydrierung. Sie erkannten, dass gute Ausbeuten an primärem Amin oft nur schwer erzielt werden können. Allerdings können die Reaktionsbedingungen die Selektivität zum primären Amin stark beeinflussen. So wird beispielsweise durch Säuren wie etwa HCl, Ammoniumsalze wie etwa NH₄Cl, oder auch durch Acetanhydrid das gebildete primäre Amin als Ammoniumsalz oder als Amid abgefangen und somit dem Angriff auf die Imin-Zwischenstufe entzogen. Eine weitere Möglichkeit zur Beeinflussung der Selektivität besteht in der Zugabe von Ammoniak. Ammoniak addiert sich reversibel an die Imin-Zwischenstufe, so dass auch auf diese Art ein Angriff des primären Amins stark zurückgedrängt wird. Durch Hydrierung der geminalen Diamin-Zwischenstufe bildet sich unter Freisetzung des Ammoniaks das primäre Amin. Auch die Zugabe von Wasser als weitere Alternative führt zu einer deutlichen Reduzierung der Nebenproduktbildung und somit zu einer Erhöhung der Bildung des primären Amins. Last but not least führt auch der Zusatz von Hydroxiden oder Carbonaten zu einer Verbesserung der Selektivität zum primären Amin. Die Autoren gehen hier von einer Inhibierung der sauren Stellen am Hydrierkatalysator aus. Die Autoren lehren somit die Zugabe eines Additivs zur Erhöhung der Selektivität zum primären Amin.

Eine andere mechanistische Studie in P. Schärringer et al, J. Catal. 253 (2008), Seiten 167 bis 179 beschreibt die Hydrierung von Acetonitril an Raney-Kobalt in Gegenwart von Hexan. Bei einer bereits relativ verdünnten Fahrweise mit einer Startkonzentration von 9,52 Mol Acetonitril pro Liter betrug die Selektivität zum Diethylamin 11%. Dies zeigt, dass ohne Zusatz von Additiven der Anteil am unerwünschten sekundären Amin recht hoch ist.

EP 0,913,388 A lehrt speziell den Zusatz von wässrigem Lithiumhydroxid zur Erhöhung der Selektivität bei der Nitrilhydrierung an Raney-Kobalt.

A.R. Cartolano et al., 2004, "Amines by Reduction", Kirk-Othmer Encyclopedia of Chemical Technology, electronic release, lehren ebenfalls bei der Nitrilhydrierung eine Erhöhung der Selektivität zu primärem Amin durch den Zusatz von Wasser. Ferner wird darin auch eine Zunahme der Bildung von sekundärem und tertiärem Amin auf Kosten der Bildung von primärem Amin mit steigender Hydriertemperatur gelehrt.

Neben der allgemeinen Literatur zur Herstellung von primären Aminen durch Hydrierung der entsprechenden Nitrile gibt es im Stand der Technik auch Publikationen zur Synthese von 2,2-Difluorethylamin als spezielles Zielmolekül.

So lehren A. Donetti et al., J. Med. Chem. 1989, 32, Seiten 957 bis 961 die Synthese von 2,2-Difluorethylamin durch Reduktion von Difluoracetamid mit Diboran und nachfolgender Zugabe von gasförmigem Chlorwasserstoff zur Isolierung des 2,2-Difluorethylamin-Hydrochlorids als weiße Kristalle. Die Ausbeute des Hydrochlorids betrug 48%. R. Kluger et al., J. Am. Chem. Soc. 1982, 104, Seiten 2891 bis 2897 beschreiben ebenfalls die Reduktion von Difluoracetamid mit Diboran (aus Bortrifluorid-etherat und Natriumborhydrid), jedoch ohne nachfolgende Umsetzung mit Chlorwasserstoff. Dabei wurde 2,2-Difluorethylamin mit einer Ausbeute von 60% erhalten.

Nachteilig an beiden Synthesen ist der Einsatz von Diboran beziehungsweise einer Mischung aus Bortrifluorid-etherat und Natriumborhydrid als Reduktionsmittel, welches in der Herstellung aufwändig und relativ teuer ist. Zudem ist Diboran toxisch. Die bei der Reduktion entstehenden B-Verbindungen sowie eventuelle restliche Mengen an Diboran müssen aufwändig entsorgt werden. Zudem fällt bei der Synthese nach A. Donetti et al. das 2,2-Difluorethylamin-Hydrochlorid an, welches dann in einem weiteren Schritt durch Umsetzung mit einer Base, wie beispielsweise Natriumhydroxid, zur Gewinnung des freien 2,2-Difluorethylamins umzusetzen ist.

Einen gänzlich anderen Weg zur Synthese von 2,2-Difluorethylamin lehrt die Bayer Cropscience AG in WO 2012/062,703. Die mehrstufige Synthese geht von einem 2,2-Difluor-1-halogenethan aus und setzt dieses in der ersten Stufe in einer mehrstündigen Autoklavenreaktion mit Prop-2-en-1-amin zum N-(2,2-Difluorethyl)prop-2-en-1-amin um. Das erhaltene Reaktionsgemisch wird anschließend zur Phasentrennung mit Wasser versetzt und die organische Phase enthaltend das N-(2,2-Difluorethyl)prop-2-en-1-amin destilliert. In der zweiten Stufe wird dann das aufgereinigte N-(2,2-Difluorethyl)prop-2-en-1-amin in 2-Aminoethanol gelöst und mit Palladium 10%ig auf Aktivkohle zur Spaltung auf 90°C erhitzt. Im Vakuum kann dabei 2,2-Difluorethylamin abdestilliert werden.

Nachteilig an diesem Verfahren sind die aufwändige, zweistufige Verfahrensweise mit destillativer Zwischenreinigung, sowie die gemäß den beiden Beispielen nur geringe Ausbeute von 52% beziehungsweise 59% über beide Stufen gerechnet. Des Weiteren benötigt diese Synthese mit stöchiometrischen Mengen an 2,2-Difluor-1-halogenethan und Prop-2-en-1-amin zwei eher schwer zugängliche Einsatzstoffe.

Im Gegensatz dazu wäre Difluoracetonitril ein wesentlich leichter zugänglicher Einsatzstoff. In US 2014/0,012,033 ist beispielsweise dessen direkte Herstellung durch Umsetzung von Halodifluormethan mit Cyanid im basischen Medium beschrieben. Halodifluormethan steht beispielsweise in Form des Chlordifluormethans in größeren Mengen leicht zur Verfügung, da dieses ein gängiges Kältemittel mit der Kurzbezeichnung R22 ist.

WO 2011/069,994 offenbart eine mehrstufige Synthese ausgehend von Difluoracetonitril. In der ersten Stufe wird Difluoracetonitril in Gegenwart einer organischen Säure, einem Säurechlorid oder einem Säureanhydrid, beispielsweise Acetanhydrid, und eines Hydrierkatalysators, beispielsweise Palladium 5% auf Aktivkohle, zum entsprechenden Difluorethylacetamid hydriert. In der zweiten Stufe wird dann das gebildete Difluorethylacetamid mit einer Säure, beispielsweise Salzsäure, versetzt und zum 2,2-Difluorethylammonium-Hydrochlorid gespalten. Dieses wäre anschließend in einer dritten Stufe durch Umsetzung mit einer Base, wie beispielsweise Natriumhydroxid, zum freien 2,2-Difluorethylamin umzusetzen.

Nachteilig an diesem Verfahren sind die aufwändige, dreistufige Verfahrensweise, sowie die geringe Ausbeute von nur 69% über die ersten beiden Stufen hinweg. Ferner wird als Hilfsstoff eine organische Säure, ein Säurechlorid oder ein Säureanhydrid in überstöchiometrischer Menge benötigt, welches zur Bildung größerer Mengen an Koppelprodukt führt. Zudem wird bei der Freisetzung des 2,2-Difluorethylamins eine stöchiometrische Menge an Base benötigt, sowie eine entsprechende Menge an Salz erhalten, welches zu entsorgen ist.

Im Rahmen der vorbereitenden Arbeiten zur genannten WO 2011/069,994 wurde dabei auch festgestellt, dass das von Gilman et al. in J. Am. Chem. Soc. 1943, 65(8), Seiten 1458 bis 1460 beschriebene Verfahren zur Herstellung von 2,2,2-Trifluorethylamin durch Hydrierung von Trifluoracetonitril an Platinoxid nicht für die Hydrierung von Difluoracetonitril geeignet ist. Gemäß der Beschreibung in WO 2011/069,994 wurde in erster Linie eine Vielzahl höheralkylierter Reaktionsprodukte erhalten. 2,2-Difluorethylamin konnte nur in Spuren erhalten werden. Demnach scheint also die direkte Hydrierung von Difluoracetonitril nicht zur Darstellung von 2,2-Difluorethylamin geeignet zu sein.

Aufgabe der vorliegenden Erfindung war es, ein Verfahren zur Herstellung von 2,2-Difluorethylamin zu finden, welches die Nachteile des Standes der Technik nicht oder nur in abgeschwächter Form aufweist, insbesondere auf einem vergleichsweise leicht zugänglichen Einsatzstoff beruht und durch welches 2,2-Difluorethylamin in einfacher Art und Weise in akzeptabel hoher Ausbeute und Reinheit erhältlich ist.

Demgemäß wurde nun ein Verfahren zur Herstellung von 2,2-Difluorethylamin gefunden, bei dem man
a) als Ausgangsstoff Difluoracetonitril einsetzt und dieses
b) mit Wasserstoff
c) bei einem Wasserstoff-Partialdruck von 2 bis 25 MPa und
d) einer Temperatur von 20 bis 250°C
e) in Gegenwart eines heterogenen Katalysators enthaltend mindestens ein Element aus der Gruppe Fe, Co, Ni, Ru, Rh, Pd und Pt hydriert,
   wobei man die Hydrierung bei
f) einem Molverhältnis von Wasser zu Difluoracetonitril von 0 bis 0,1; und
g) einem Molverhältnis von Ammoniak zu Difluoracetonitril von 0 bis 1 durchführt.

Basierend auf der Lehre des Standes der Technik in WO 2011/069,994, nach der die direkte Hydrierung von Difluoracetonitril nicht zur Darstellung von 2,2-Difluorethylamin aufgrund der Bildung einer Vielzahl höheralkylierter Reaktionsprodukte und nur von Spuren an 2,2-Difluorethylamin geeignet sei, war es vollkommen unerwartet und überraschend, dass es unter ganz bestimmten Bedingungen doch möglich ist, 2,2-Difluorethylamin in einstufiger Fahrweise direkt durch Hydrierung von Difluoracetonitril in hoher Ausbeute und Reinheit zu gewinnen.

Difluoracetonitril ist aufgrund seiner guten Zugänglichkeit ein besonders vorteilhafter Einsatzstoff. Wie eingangs bereits beschrieben, kann Difluoracetonitril beispielsweise direkt durch Umsetzung von Halodifluormethan mit Cyanid im basischen Medium erhalten werden (siehe US 2014/0,012,033). Insbesondere Chlordifluormethan als gängiges Kältemittel mit der Kurzbezeichnung R22 steht in größeren Mengen zur Verfügung. Chlordifluormethan kann beispielsweise aus Trichlormethan (Chloroform) durch partielle Fluoridierung in flüssiger Phase in Gegenwart von Antimon(V)-chloridfluorid als Katalysator gewonnen werden.

Des Weiteren kann Difluoracetonitril leicht durch Entwässerung von Difluoracetamid in Gegenwart von Entwässerungsmitteln, wie beispielsweise Phosphorpentoxid, erhalten werden (siehe F. Swarts, "Sur les nitriles des acides fluor- et difluoracetiques" in Bull. Soc. Chim. Belg. 1922, 31, 364-365).

Um negative Auswirkungen auf die Aktivität der eingesetzten Katalysatoren zu vermeiden oder zumindest zu verringern, weist das einzusetzende Difluoracetonitril bevorzugt einen Fluoridgehalt von ≤ 10 Gew.-ppm und einen Cyanidgehalt von ≤ 100 Gew.-ppm auf. Besonders bevorzugt weist das 2,2-Difluorethylamin einen Fluoridgehalt von ≤ 5 Gew.-ppm und ganz besonders bevorzugt von ≤ 2 Gew.-ppm auf. Der Cyanidgehalt beträgt besonders bevorzugt ≤ 50 Gew.-ppm, ganz besonders bevorzugt ≤ 25 Gew.-ppm und insbesondere ≤ 10 Gew.-ppm.

Der beim erfindungsgemäßen Verfahren einzusetzende Wasserstoff kann beispielsweise in reiner Form oder auch im Gemisch mit weiteren Gasen eingesetzt werden. Im Allgemeinen setzt man aus Praktikabilitätsgründen technisch reinen Wasserstoff ein. Wird der Wasserstoff in Form eines Wasserstoff enthaltenden Gases eingesetzt, so enthält dieses üblicherweise Beimengungen von anderen Inertgasen, wie etwa Stickstoff, Helium, Neon, Argon oder Kohlendioxid. Als Wasserstoff enthaltende Gase können beispielsweise Reformerabgase, Raffineriegase und dergleichen eingesetzt werden, sofern diese Gase keine Kontaktgifte für die eingesetzten Hydrierkatalysatoren, wie zum Beispiel Kohlenmonoxid, enthalten. Bevorzugt wird jedoch reiner Wasserstoff beziehungsweise im Wesentlichen reiner Wasserstoff im Verfahren eingesetzt. Dieser besitzt üblicherweise einen Gehalt von ≥ 99 Gew.-% Wasserstoff, bevorzugt von ≥ 99,9 Gew.-% Wasserstoff, besonders bevorzugt von ≥ 99,99 Gew.-% Wasserstoff und insbesondere bevorzugt von ≥ 99,999 Gew.-% Wasserstoff.

Die erfindungsgemäße Hydrierung von Difluoracetonitril erfolgt in Gegenwart eines heterogenen Katalysators enthaltend mindestens ein Element aus der Gruppe Fe, Co, Ni, Ru, Rh, Pd und Pt. Es können jedoch auch Katalysatoren auf Basis von Osmium oder Iridium eingesetzt werden. Bevorzugt enthalten die heterogenen Katalysatoren mindestens ein Element aus der Gruppe Co, Ni und Ru, besonders bevorzugt aus der Gruppe Co und Ni und insbesondere Co. Die Katalysatoren können natürlich auch noch weitere Elemente als Promotoren enthalten.

Es können dabei beispielsweise sogenannte Vollkatalysatoren, geträgerte Katalysatoren oder Raney-Katalysatoren eingesetzt werden. Je nach gewünschter Verfahrensführung (Suspensionshydrierung, Wirbelschichthydrierung, Festbetthydrierung) kann der Katalysator beispielsweise als Pulver, Splitt oder Formkörper eingesetzt werden.

Werden Vollkatalysatoren eingesetzt, so beträgt der Gehalt an den Elementen Fe, Co, Ni, Ru, Rh, Pd und Pt, bezogen auf die Gesamtmasse des Katalysators, bevorzugt 30 bis 100 Gew.-%, besonders bevorzugt 50 bis 99 Gew.-% und ganz besonders bevorzugt 55 bis 98 Gew.-%. Bei geträgerten Katalysatoren beträgt der Gehalt an den Elementen Fe, Co, Ni, Ru, Rh, Pd und Pt bezogen auf die Gesamtmasse des Katalysators bevorzugt 0,01 bis 30 Gew.-%, wobei im Falle der Edelmetalle Ru, Rh, Pd und Pt deren Gehalt eher im Bereich 0,01 bis 10 Gew.-%, bevorzugt 0,05 bis 5 Gew.-%, und im Falle der restlichen Metalle deren Gehalt eher im Bereich 1 bis 30 Gew.-%, bevorzugt 5 bis 20 Gew.-% liegt.

Werden oxidische Katalysatoren eingesetzt, so werden diese üblicherweise vor dem Einsatz außerhalb des Reaktors oder im Reaktor durch Reduktion der Metalloxide in einem Wasserstoff-enthaltenden Gasstrom bei erhöhter Temperatur aktiviert. Sofern die Katalysatoren außerhalb des Reaktors reduziert werden, kann eine anschließende Passivierung durch einen Sauerstoff-enthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Raney-Katalysatoren dar, die durch Laugung mit wässriger Base, wie zum Beispiel in EP 1,209,146 A1 beschrieben, aktiviert werden können.

Werden beim erfindungsgemäßen Verfahren Festbettkatalysatoren eingesetzt, also Katalysatoren, die im Reaktor fest angeordnet sind, wie dies beispielsweise bei Vollkatalysatoren oder geträgerten Katalysatoren möglich ist, so werden bevorzugt die in EP 0,742,045 A1 offenbarten, und mit Phosphor, Mangan und Alkalimetallen dotierten, Kobalt-haltigen Vollkatalysatoren eingesetzt. Die katalytisch aktive Masse dieser Katalysatoren umfasst vor der reduktiven Aktivierung bevorzugt 55 bis 98 Gew.-% und insbesondere 75 bis 95 Gew.-% Kobalt, sowie bevorzugt 0,2 bis 15 Gew.-% Phosphor, bevorzugt 0,2 bis 15 Gew.-% Mangan und bevorzugt 0,05 bis 5 Gew.-% Alkalimetall, insbesondere Natrium, jeweils berechnet als Oxid.

Weitere geeignete Katalysatoren für die Festbettfahrweise sind beispielsweise auch die in EP 0,963,975 A1 offenbarten Katalysatoren, deren katalytisch aktive Masse vor der Aktivierung mit Wasserstoff bevorzugt 22 bis 40 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ beziehungsweise MnO₂, und keine sauerstoffhaltigen Verbindungen des Molybdäns enthält.

Werden geträgerte Katalysatoren eingesetzt, so werden bevorzugt Al₂O₃, SiO₂, ZrO₂ oder TiO₂ als Träger verwendet.

Die beim erfindungsgemäßen Verfahren bevorzugten Katalysatoren sind jedoch Raney-Katalysatoren enthaltend Nickel und/oder Kobalt, insbesondere Raney-Katalysatoren enthaltend Kobalt. Raney-Katalysatoren werden auch als sogenannte Skelett-Katalysatoren bezeichnet. Bei diesen wird der aktive Katalysator als eine Art Metallschwamm aus einer Ni und/oder Co-haltigen Legierung mit einem Gerüstbildner wie etwa Aluminium oder Silizium durch Herauslösen des Gerüstbildners mit Säure oder Lauge hergestellt. Bevorzugt wird Aluminium als Gerüstbildner eingesetzt. Bevorzugt werden die Gerüstbildner mit Alkalilauge, vorzugsweise Natronlauge, herausgelöst. Reste des ursprünglichen Gerüstbildners können zuweilen auch synergetisch wirken und somit die katalytischen Eigenschaften verbessern. Nach dem Herauslösen des Gerüstbildners werden die Raney-Katalysatoren in der Regel mit Wasser oder einem organischen Lösungsmittel gewaschen.

Die Raney-Katalysatoren können auch über einzelne oder mehrere weitere Elemente als Promotoren dotiert sein. Beispiele für mögliche Promotoren sind Metalle der Nebengruppen IB, VIB und/oder VIII des Periodensystems, wie beispielsweise Chrom, Eisen, Molybdän, Nickel oder Kupfer. Werden dotierte Raney-Katalysatoren eingesetzt, so sind diese bevorzugt mit Chrom dotiert.

Die Aktivierung der Raney-Katalysatoren durch Auslaugen des löslichen Gerüstbildners kann entweder im Reaktor selbst oder vor der Befüllung des Reaktors erfolgen. Da die voraktivierten Katalysatoren luftempfindlich und pyrophor sind, werden diese bevorzugt in der Regel unter einem Medium wie zum Beispiel Wasser, einem organischen Lösungsmittel oder dem Difluoracetonitril aufbewahrt.

Die beim erfindungsgemäßen Verfahren bevorzugten Raney-Katalysatoren enthalten typischerweise neben Nickel beziehungsweise Kobalt noch 1 bis 30 Gew.-% Al, bevorzugt 2 bis 12 Gew.-% Al, besonders bevorzugt 3 bis 6 Gew.-% Al, 0 bis 10 Gew.-% Cr, bevorzugt 0,1 bis 7 Gew.-% Cr, besonders bevorzugt 0,5 bis 5 Gew.-% Cr, insbesondere 1,5 bis 3,5 Gew.-% Cr, 0 bis 10 Gew.-% Fe, bevorzugt 0,1 bis 3 Gew.-% Fe, besonders bevorzugt 0,2 bis 1 Gew.-% Fe. Zudem enthalten die bevorzugt einzusetzenden Kobalt-haltigen Raney-Katalysatoren üblicherweise 0 bis 10 Gew.-% Ni, bevorzugt 0,1 bis 7 Gew.-% Ni, besonders bevorzugt 0,5 bis 5 Gew.-% Ni und insbesondere 1 bis 4 Gew.-% Ni, wobei die Gewichtsangaben jeweils auf das Katalysatorgesamtgewicht bezogen sind.

Ganz besonders bevorzugt ist beim erfindungsgemäßen Verfahren der Einsatz eines Kobalt-haltigen Raney-Katalysators der Zusammensetzung 2 bis 6 Gew.-% Al, >86 Gew.-% Co, 0 bis 1 Gew.-% Fe, 1 bis 4 Gew.-% Ni und 1,5 bis 3,5 Gew.-% Cr. Ein derartiger Katalysator wird beispielsweise von der Firma W. R. Grace unter der Bezeichnung "Raney 2724" angeboten.

Die Katalysatoren können gegebenenfalls bei nachlassender Aktivität und/oder Selektivität mit den dem Fachmann bekannten Methoden regeneriert werden. Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierlich entnommen, ex situ regeneriert und zurückgeführt.

Das erfindungsgemäße Verfahren wird bei einem Wasserstoff-Partialdruck von 2 bis 25 MPa abs durchgeführt. Der Wasserstoff-Partialdruck beträgt bevorzugt ≥ 4 MPa abs, besonders bevorzugt ≥ 5 MPa abs und ganz besonders bevorzugt ≥ 6 MPa abs, sowie bevorzugt ≤ 20 MPa abs, besonders bevorzugt ≤ 18 MPa abs und ganz besonders bevorzugt ≤ 15 MPa abs.

Die Temperatur, bei der das erfindungsgemäße Verfahren durchgeführt wird, liegt bei 20 bis 250°C.

Das erfindungsgemäße Verfahren kann diskontinuierlich, halbkontinuierlich oder kontinuierlich durchgeführt werden. Bei der diskontinuierlichen Fahrweise, auch Batch-Fahrweise genannt, werden Katalysator und die gesamte Menge an Difluoracetonitril vorgelegt und mit Wasserstoff auf den gewünschten Reaktionsdruck aufgepresst, wobei verbrauchter Wasserstoff gegebenenfalls nachgeliefert wird. Bei der halbkontinuierlichen Fahrweise, auch Semi-Batch-Fahrweise genannt, werden Katalysator und gegebenenfalls eine Teilmenge an Difluoracetonitril vorgelegt und anschließend Wasserstoff beim gewünschten Reaktionsdruck sowie weiteres Difluoracetonitril kontinuierlich nachdosiert. Die nachzudosierende Menge ist hier natürlich begrenzt durch die Aufnahmekapazität des Reaktors. Bei der kontinuierlichen Fahrweise werden Katalysator und gegebenenfalls eine Teilmenge an Difluoracetonitril vorgelegt und anschließend Wasserstoff beim gewünschten Reaktionsdruck sowie weiteres Difluoracetonitril kontinuierlich nachdosiert, wobei gleichzeitig eine entsprechende Menge an Reaktionsgemisch kontinuierlich entnommen wird. Es sind natürlich auch Kombinationen aus den genannten Verfahrensvarianten denkbar und möglich.

In einer bevorzugten Ausführungsform der halbkontinuierlichen oder kontinuierlichen Fahrweise wird Difluoracetonitril in einer Menge kontinuierlich zugeführt, die nicht größer ist als die Menge an Difluoracetonitril, die mit dem ebenfalls zugeführten Wasserstoff hydriert wird. Die sogenannte Zuführrate an Difluoracetonitril ist bevorzugt so einzustellen, dass ein relativ hoher, bevorzugt quasi Vollumsatz erreicht wird. Die Hydriergeschwindigkeit des Difluoracetonitrils wird beispielsweise durch die eingestellte Temperatur, den vorliegenden Wasserstoff-Partialdruck, die Art des Gemisches wie etwa der Konzentrationen der einzelnen Komponenten beziehungsweise möglicher Zusatzstoffe wie etwa Lösungsmittel, der Menge und Art des Katalysators, die Durchmischungsgüte des Reaktorinhalts, oder auch durch die Verweilzeit beeinflusst.

Bevorzugt wird das erfindungsgemäße Verfahren in halbkontinuierlicher Fahrweise durchgeführt.

Als Reaktoren können prinzipiell alle Reaktoren eingesetzt werden, die für die Durchführung heterogen katalysierter, exothermer Gas-Flüssig-Reaktionen unter erhöhtem Druck geeignet sind. Darin eingeschlossen sind Reaktoren zur Festbettfahrweise, zur Wirbelschichtfahrweise sowie zur Suspensionsfahrweise. Geeignete Reaktoren sind dabei beispielsweise Schachtreaktoren, Rohrreaktoren, Rohrbündelreaktoren, Wirbelschichtreaktoren, Rührkessel, Strahlschlaufenreaktoren, Strahldüsenreaktoren, Blasensäulenreaktoren sowie mehrere gleiche oder unterschiedliche Reaktoren in Kaskadenschaltung.

Im Falle eines Festbettreaktors kann das Verfahren in Sumpf- oder Rieselfahrweise betrieben werden. Durch den bevorzugten Einsatz von Raney-Katalysatoren sind Reaktoren bevorzugt, die eine Suspensionsfahrweise ermöglichen und daher eine gewisse Vermischung sicherstellen. Als mögliche Beispiele hierzu seien Wirbelschichtreaktoren, Rührkessel, Strahlschlaufenreaktoren, Strahldüsenreaktoren und Blasensäulenreaktoren genannt.

Die Reaktoren sind vorteilhafterweise aus einem beständigen, korrosionsresistenten Material. Üblicherweise werden daher Legierungen auf Basis von Mo, Cr, Co, Fe, Cu, Mn, Ti, Zr, Al, C und W eingesetzt, wie beispielsweise Hastelloy® oder Inconel®. Auch Edelstähle, wie beispielsweise mit den Werkstoffnummern 304, 304L, 316 oder 316L, sind in der Regel gut geeignet.

Die Hydrierung der Nitrilgruppen im Difluoracetonitril findet unter Freisetzung von Wärme statt, die in der Regel abgeführt werden muss. Die Wärmeabfuhr kann durch eingebaute Wärmeüberträgerflächen, Kühlmantel oder außenliegende Wärmeüberträger in einem Umlaufkreis um den Reaktor erfolgen. Der Hydrierreaktor beziehungsweise die Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeüberträgers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist so eine einfache und kostengünstige Bauform möglich. Eine Alternative stellt, im Falle einer Festbettfahrweise, ein gekühlter Rohrbündelreaktor dar. Auch eine Kombination verschiedener Fahrweisen ist denkbar.

Wie bei Suspensionsfahrweisen üblich, kann der suspendierte Katalysator in der Regel durch Sedimentation und/oder Filtration mittels einer Filtervorrichtung im Reaktor zurückgehalten werden.

Die Menge an Katalysator im Verhältnis zur Menge an Difluoracetonitril bei diskontinuierlicher Fahrweise beziehungsweise im Verhältnis zur Menge an Difluoracetonitril pro Zeiteinheit bei halbkontinuierlicher oder kontinuierlicher Fahrweise ist letztendlich abhängig von der gewünschten Hydriergeschwindigkeit des Difluoracetonitrils unter den vorliegenden Bedingungen wie etwa Wasserstoff-Partialdruck, Temperatur, Konzentration an Difluoracetonitril und dergleichen. Je nach gewünschter Hydriergeschwindigkeit kann die erforderliche Menge an Katalysator vom Fachmann in einfachen Routineversuchen leicht ermittelt werden. In der Regel setzt man beim erfindungsgemäßen Verfahren bei diskontinuierlicher beziehungsweise halbkontinuierlicher Fahrweise 0,1 bis 30 Gew.-% Katalysator bezogen auf die Menge an Difluoracetonitril ein. Bei kontinuierlicher Fahrweise führt man das erfindungsgemäße Verfahren im Allgemeinen bei einer Belastung von 0,01 bis 5 kg Difluoracetonitril pro kg Katalysator und Stunde durch.

Die erforderliche Reaktionszeit wird maßgebend von der Hydriergeschwindigkeit bestimmt. Je größer die Hydriergeschwindigkeit, desto kürzer die erforderliche Reaktionszeit. Auch die erforderliche Reaktionszeit kann vom Fachmann in einfachen Routineversuchen leicht ermittelt werden. In der Regel wird man aus rein praktischen Gründen bei diskontinuierlicher oder halbkontinuierlicher Fahrweise durch Temperatur, Wasserstoff-Partialdruck, Art des Gemisches wie etwa der Konzentrationen der einzelnen Komponenten beziehungsweise möglicher Zusatzstoffe wie etwa Lösungsmittel, Menge und Art des Katalysators und Durchmischungsgüte des Reaktorinhalts die Reaktionszeit so einstellen, dass das Reaktionsgemisch nach wenigen Sekunden bis mehreren Tagen aufgearbeitet werden kann, bevorzugt nach 1 Minute bis 10 Tagen.

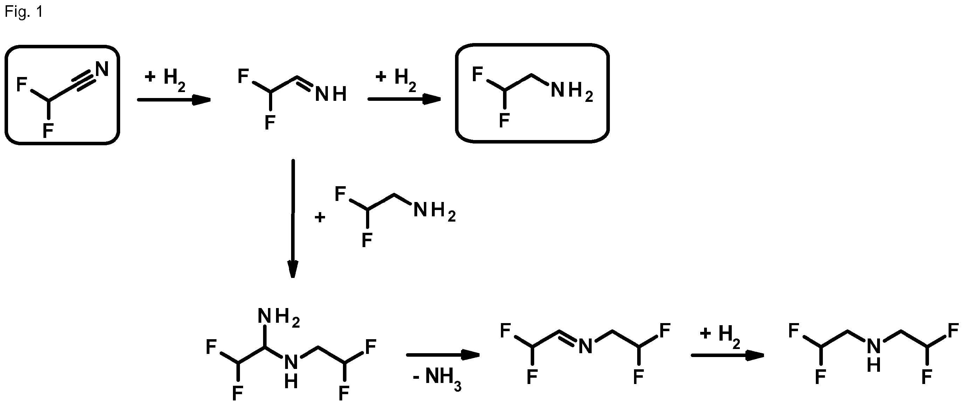

Bei der Hydrierung von Nitrilen zu primären Aminen konkurriert die Bildung des primären Amins mit der Bildung sekundärer Amine. In der Literatur allgemein anerkannt ist dabei ein Reaktionsmechanismus, nach dem sich aus der Nitrilgruppe durch Hydrierung zunächst eine Imingruppe bildet und diese dann in einem zweiten Schritt durch weitere Hydrierung in eine primäre Amingruppe überführt wird. Durch eine Addition von bereits gebildetem primärem Amin an die Imin-Zwischenstufe, nachfolgender Abspaltung von Ammoniak und Hydrierung des dadurch gebildeten Imins entsteht dabei ein sekundäres Amin. Die Reaktionsmechanismen sind beispielsweise aus S. Gomez et al. in Adv. Synth. Catal. 2002, 344, No. 10, Seiten 1037 bis 1057 zu entnehmen. Für die Hydrierung von Difluoracetonitril sind die Hauptreaktion und die Nebenreaktion zum sekundären Amin in Abbildung 1 dargestellt.

Gemäß der Lehre aus dem Stand der Technik, wie beispielsweise S. Gomez et al. in Adv. Synth. Catal. 2002, 344, No. 10, Seiten 1037 bis 1057 oder A.R. Cartolano et al., 2004, "Amines by Reduction", Kirk-Othmer Encyclopedia of Chemical Technology, electronic release, werden die Nebenreaktionen durch Zugabe diverser Additive deutlich zurückgedrängt und somit die Selektivität zum primären Amin und dessen Ausbeute deutlich erhöht. Als selektivitätsfördernde Additive sind insbesondere Säuren, Acetanhydrid, Ammoniak, Ammoniumsalze aus Ammoniak und Säuren, Wasser, sowie Hydroxide und Karbonate genannt. Insbesondere Wasser und Ammoniak sind dabei sehr gängige Additive zur Unterdrückung der Nebenreaktion und somit zur Erhöhung der Selektivität zum primären Amin. Entsprechend wäre auch zu erwarten gewesen, dass diese Additive auch bei der Hydrierung von Difluoracetonitril zu 2,2-Difluorethylamin eine positive Wirkung zeigen. Erstaunlicherweise konnte jedoch erfindungsgemäß festgestellt werden, dass gerade diese Additive die Selektivität zum 2,2-Difluorethylamin deutlich verringern und somit auch dessen Ausbeute signifikant verschlechtern. So konnte völlig überraschend gezeigt werden, dass gerade bei Abwesenheit dieser Additive beziehungsweise bei nur geringen vorhandenen Mengen die Selektivität zum 2,2-Difluorethylamin besonders hoch ist.

Daher führt man das erfindungsgemäße Verfahren bei
f) einem Molverhältnis von Wasser zu Difluoracetonitril von 0 bis 0,2; und
g) einem Molverhältnis von Ammoniak zu Difluoracetonitril von 0 bis 1
durch.

Bevorzugt beträgt das Molverhältnis von Wasser zu Difluoracetonitril 0 bis 0,1, besonders bevorzugt 0 bis 0,05, ganz besonders bevorzugt 0 bis 0,02 und insbesondere 0,01.

Bevorzugt beträgt das Molverhältnis von Ammoniak zu Difluoracetonitril 0 bis 0,5, besonders bevorzugt 0 bis 0,2 und ganz besonders bevorzugt 0 bis 0,1, insbesondere 0 bis 0,05.

Bevorzugt vermeidet man beim erfindungsgemäßen Verfahren auch die Gegenwart größerer Mengen an organischen oder anorganischen Säuren sowie derer Anhydride. Bevorzugt beträgt das Molverhältnis von organischen und anorganischen Säuren sowie derer Anhydride zu Difluoracetonitril in Summe 0 bis 0,5, besonders bevorzugt 0 bis 0,25, ganz besonders bevorzugt 0 bis 0,1 und insbesondere 0 bis 0,05.

Ebenfalls vermeidet man beim erfindungsgemäßen Verfahren bevorzugt auch die Gegenwart größerer Mengen an Alkali- und Erdalkali-Hydroxiden und -Karbonaten. Bevorzugt beträgt das Molverhältnis von Alkali- und Erdalkali-Hydroxiden und Karbonaten zu Difluoracetonitril in Summe 0 bis 0,1, besonders bevorzugt 0 bis 0,05, ganz besonders bevorzugt 0 bis 0,02 und insbesondere 0 bis 0,01.

A.R. Cartolano et al., 2004, "Amines by Reduction", Kirk-Othmer Encyclopedia of Chemical Technology, electronic release, lehren, dass mit steigender Temperatur in der Hydrierung von Nitrilen zu Aminen die Nebenreaktion zu sekundären und tertiären Aminen zunimmt und somit die Ausbeute an primärem Amin abnimmt. Entsprechend wäre daher zu erwarten gewesen, dass auch bei der Hydrierung von Difluoracetonitril zum 2,2-Difluorethylamin eher niedrigere Temperaturen zu einer höheren 2,2-Difluorethylamin-Ausbeute führen. Erstaunlicherweise konnte jedoch erfindungsgemäß festgestellt werden, dass gerade dies nicht der Fall ist, sondern gerade andersherum eine eher höhere Temperatur zu einer besseren Selektivität und höheren Ausbeute an 2,2-Difluorethylamin führt. Daher führt man das erfindungsgemäße Verfahren bevorzugt bei einer Temperatur von ≥ 50°C, besonders bevorzugt von ≥ 100°C und ganz besonders bevorzugt von ≥ 120°C, sowie bevorzugt von ≤ 220°C, besonders bevorzugt von ≤ 200 und ganz besonders bevorzugt von ≤ 180°C durch. Dementsprechend bevorzugt ist daher ein Bereich von 50 bis 220°C und ganz besonders bevorzugt von 120 bis 180°C.

Das erfindungsgemäße Verfahren kann auch in Gegenwart von Lösungsmittel durchgeführt werden. Vorteilhafterweise sollten die einzusetzenden Lösungsmittel unter den Reaktionsbedingungen stabil und inert sein.

Im Rahmen der Erfindung wurde dabei überraschend festgestellt, dass Lösungsmittel mit einer bestimmten Mindestpolarität die Selektivität zum gewünschten 2,2-Difluorethylamin deutlich erhöhen und somit die Ausbeute verbessern. Als besonders vorteilhaft hat sich dabei herausgestellt, die Hydrierung in Gegenwart eines aprotischen Lösungsmittels, welches bei 25°C eine relative Permittivität εᵣ von ≥ 2 aufweist, durchzuführen.

Geeignete Klassen aprotischer Lösungsmittel sind etwa Ether, aromatische Kohlenwasserstoffe, Lactame und die bei der Hydrierung des Difluoracetonitrils gebildeten höheren Amine, welche eine relative Permittivität εᵣ von ≥ 2 (bei 25°C) aufweisen. Als Beispiele möglicher Ether seien lineare und zyklische Ether, aliphatische, aromatische und aliphatisch-aromatischeEther als auch Monoether, Oligoether und Polyether genannt, wie beispielsweise Dimethylether, Diethylether, Di-n-propylether, Diisopropylether, Di-n-butylether, Diisobutylether, Diisoamylether, Ethylpropylether, Isopropylethylether, Methyl-tert-butylether, Cyclohexylmethylether, 1,2-Dimethoxyethan, Anisol (Methylphenylether), Phenetol (Ethylphenylether), Diphenylether, Dipropylenglykol-dimethylether, 2-(2-Ethoxyethoxy)-2-methylpropan, Diethylenglykol-di-n-butylether, Diethylenglykol-dimethylether, Diethylenglykol-diethylether, Triethylenglykol-dimethylether, Tetraethylenglykol-dimethylether, Tetrahydrofuran, Methyl-tetrahydrofuran, 1,4-Dioxan, 1,3-Dioxolan und Polyether des Ethylenoxids und/oder Propylenoxids, wie etwa Poly(ethylenglykol)-dimethylether oder hoch-ethoxylierte Diether eines höhermolekularen Alkohols mit einem Gesamtmolgewicht von über 400 g/mol. Als Beispiele möglicher aromatischer Kohlenwasserstoffe seien Toluol und Xylole genannt. Als Beispiele möglicher Lactame seien N-Alkyl-substituierte Pyrrolidone, Piperidone und ε-Caprolactame wie beispielsweise N-Methylpyrrolidon, N-Ethylpyrrolidon, N-Methylpiperidon, N-Ethylpiperidon, N-Methyl-ε-caprolactam und N-Ethyl-ε-caprolactam genannt. Als bei der Hydrierung des Difluoracetonitrils gebildete höhere Amine und Nebenprodukte seien beispielsweise Bis-(2,2-difluorethyl)amin und Tri-(2,2-difluorethyl)amin genannt.

Hinsichtlich einer hohen Selektivität und hohen Ausbeute besonders vorteilhaft sind aprotische Lösungsmittel mit einer relativen Permittivität εᵣ von ≥ 5 (bei 25°C).

Für die nachfolgende destillative Aufarbeitung des Reaktionsgemisches ist es vorteilhaft, wenn sich der Siedepunkt der eingesetzten Lösungsmittels um mindestens 2°C, bevorzugt um mindestens 5°C vom Siedepunkt des 2,2-Difluorethylamins unterscheidet. Die Lösungsmittel können dabei entweder einen niedrigeren oder höheren Siedepunkt aufweisen.

Besonders vorteilhaft sind entsprechende Lösungsmittel, welche einen höheren Siedepunkt als 2,2-Difluorethylamin aufweisen, um bei einer destillativen Aufarbeitung des Reaktionsgemisches das 2,2-Difluorethylamin als Leichtsieder über Kopf oder als Seitenstrom möglichst rein gewinnen zu können. Der Siedepunkt von 2,2-Difluorethylamin liegt gemäß Sicherheitsdatenblatt von Sigma-Aldrich bei 1013,25 hPa bei 66-67°C. Die favorisierten Lösungsmittel weisen daher bei einem Druck von 1013,25 hPa abs einen Siedepunkt von ≥ 69°C, bevorzugt von ≥ 72°C, besonders bevorzugt von ≥ 75°C, ganz besonders bevorzugt von ≥ 80°C und insbesondere von ≥ 90°C auf.

Dementsprechend besonders favorisierte Lösungsmittel sind Di-n-propylether, Diisopropylether, Di-n-butylether, Diisobutylether, Diisoamylether, Ethylpropylether, Isopropylethylether, Methyl-tert-butylether, Cyclohexylmethylether, 1,2-Dimethoxyethan, Anisol (Methylphenylether), Phenetol (Ethylphenylether), Diphenylether, Dipropylenglykol-dimethylether, 2-(2-Ethoxyethoxy)-2-methylpropan, Diethylenglykol-di-n-butylether, Diethylenglykol-dimethylether, Diethylenglykol-diethylether, Triethylenglykol-dimethylether, Tetraethylenglykol-dimethylether, Methyltetrahydrofuran, 1,4-Dioxan, 1,3-Dioxolan, Poly(ethylenglykol)-dimethylether, ethoxylierte Diether eines höhermolekularen Alkohols mit einem Gesamtmolgewicht von über 400 g/mol, Toluol, Xylole, N-Methylpyrrolidon, N-Ethylpyrrolidon, N-Methylpiperidon, N-Ethylpiperidon, N-Methyl-ε-caprolactam und/oder N-Ethyl-ε-caprolactam.

Es können selbstverständlich auch mehrere Lösungsmittel gleichzeitig eingesetzt werden.

Führt man das erfindungsgemäße Verfahren in Gegenwart eines aprotischen Lösungsmittels durch, so setzt man im Allgemeinen eine Menge von 0,01 bis 100 g pro g Difluoracetonitril und bevorzugt von 0,1 bis 100 g pro g Difluoracetonitril ein.

Falls ein entsprechendes aprotisches Lösungsmittel eingesetzt wird, ist es vorteilhaft, dieses oder zumindest einen Teil davon zum Difluoracetonitril-Einsatzstoff zuzugeben und dann die Lösung des somit verdünnten Difluoracetonitrils dem Reaktor zuzuführen. Bei halbkontinuierlicher Fahrweise ist es vorteilhaft, einen Teil des entsprechenden aprotischen Lösungsmittels zusammen mit dem Katalysator im Reaktor vorzulegen und das Difluoracetonitril oder eine Lösung im anderen Teil des Lösungsmittels zuzudosieren.

Nach erfolgter Hydrierung wird das Reaktionsgemisch bevorzugt destillativ aufgearbeitet. Hierzu wird zunächst der Katalysator abgetrennt. Im Falle eines Festbettverfahrens erfolgt die Abtrennung automatisch durch die Entnahme des Reaktionsgemischs aus dem Festbettreaktor. Bei Suspensionsfahrweise wird der suspendierte Katalysator üblicherweise durch eine Filtervorrichtung im Reaktor zurückgehalten.

Die destillative Aufarbeitung kann mit dem üblichen Können eines Fachmanns durchgeführt werden. Zunächst werden dabei die verschiedenen Leichtsieder abgetrennt, die einen Siedepunkt unterhalb von 2,2-Difluorethylamin aufweisen, wie beispielsweise restlicher Wasserstoff, gegebenenfalls durch Nebenreaktion gebildeter Ammoniak oder nicht umgesetztes Difluoracetonitril. Abgetrenntes Difluoracetonitril kann beispielsweise erneut in der Hydrierung eingesetzt werden. Als nächste Fraktion kann dann bereits das 2,2-Difluorethylamin gewonnen werden. Die sogenannten Schwersieder enthalten dann höher siedende Nebenprodukte sowie eventuell eingesetztes Lösungsmittel. Die Schwersieder können beispielsweise in einem weiteren Schritt ebenfalls destillativ zur Rückgewinnung eines eventuell eingesetzten Lösungsmittels weiter aufgearbeitet werden.

Die Lösungsmittel können im Allgemeinen rückgeführt und somit ohne größeren Aufwand wiederverwendet werden.

2,2-Difluorethylamin ist somit in einer Reinheit von ≥ 95 Gew.-% erhältlich.

Abhängig von den Reaktionsbedingungen kann eine Selektivität zum 2,2-Difluorethylamin von > 80% erreicht werden. In Verbindung mit einem Difluoracetonitril-Umsatz von bis zu 100% ergibt sich somit eine Ausbeute an 2,2-Difluorethylamin von > 80%.

In einer allgemeinen Ausführungsform zur halbkontinuierlichen Herstellung von 2,2-Diflourethylamin durch Hydrierung von Difluoracetonitril an einem Raney-Cobalt-Katalysator wird dieser unter Schutzgasatmosphäre, gegebenenfalls zusammen mit einem aprotischen Lösungsmittel in einem Rührreaktor (Autoklav) vorgelegt. Danach wird der Rührreaktor unter Rühren auf die gewünschte Reaktionstemperatur aufgeheizt und Wasserstoff bis zum gewünschten Druck aufgepresst. Nun wird unter weiterem Rühren mit der kontinuierlichen Zugabe von Difluoracetonitril, welches gegebenenfalls zuvor in einem aprotischen Lösungsmittel gelöst wurde, begonnen. Der verbrauchte Wasserstoff wird dabei üblicherweise nachgepresst. Nachdem die gewünschte Menge an Difluoracetonitril über den gewünschten Zeitraum hinweg zugegeben wurde, wird der Rührreaktor zur Nachreaktion noch für kurze Zeit weiter unter den Reaktionsbedingungen belassen und dann anschließend abgekühlt und entspannt. Das Reaktionsgemisch wird dann zur destillativen Aufarbeitung einer Destillationskolonne zugeführt und 2,2-Difluorethylamin destillativ, bevorzugt als Kopf- oder Seitenstrom gewonnen.

Das erfindungsgemäße Verfahren ermöglicht die Herstellung von 2,2-Difluorethylamin, welches die Nachteile des Standes der Technik nicht oder nur in abgeschwächter Form aufweist. Es basiert auf dem Einsatz von Difluoracetonitril als vergleichsweise leicht zugänglichem Einsatzstoff und ist als Nitrilhydrierung in einfacher Art und Weise durchführbar. Es ermöglicht die Gewinnung von 2,2-Difluorethylamin in hoher Ausbeute von > 80% und Reinheit von ≥ 95 Gew.-%.

Auch wenn bei alternativen Syntheserouten für 2,2-Difluorethylamin gegebenenfalls ähnliche oder vielleicht sogar geringfügig höhere Ausbeuten erzielt werden können, so sind diese von der Verfahrensführung her komplexer und bedürfen einer mehrstufigen Synthese mit diversen Additiven und Hilfsstoffen, die anschließend wieder abzutrennen sind. Das erfindungsgemäße Verfahren besticht durch seine sehr einfache Fahrweise der Nitrilhydrierung in einer Einstufenreaktion und der sehr einfachen destillativen Aufarbeitung ohne Notwendigkeit, Additive oder Hilfsstoffe hinzuzufügen, wenngleich auch die Zugabe von bestimmten, einfach abzutrennenden Lösungsmitteln vorteilhaft ist.

### Beispiele

In allen Beispielen wurde Difluoracetonitril mit einem Fluoridgehalt von ≤ 10 Gew.-ppm, einem Wassergehalt von ≤ 10 Gew.-ppm und einem Cyanidgehalt von ≤ 100 Gew.-ppm eingesetzt.

### Katalysator A

Durch Auflösen von Kobaltnitrat, Mangannitrat und Phosphorsäure in Wasser wurde eine Lösung hergestellt, die 10 Gew.-% Kobalt, 0,55 Gew.-% Mangan und 0,45 Gew.-% H₃PO₄ enthält. Durch Zugabe einer 20%igen Natriumcarbonatlösung wurde bei einer Temperatur von 50°C gefällt. Der entstandene Niederschlag wurde gewaschen, bis im Waschwasser kein Natrium oder Nitrat mehr nachweisbar war. Der so erhaltene Feststoff wurde mit Wasser angemaischt und in einem Sprühturm versprüht (Eingangstemperatur 550°C). Das Sprühgut wurde bei 500°C getrocknet, gekollert und im Extruder zu Strängen von 4 mm Durchmesser verformt. Die Stränge wurden bei 100 bis 120°C getrocknet und anschließend 1 Stunde bei 650°C und danach 3 Stunden bei 850°C kalziniert.

Der so hergestellte Katalysator enthielt 90,4 Gew.-% Kobalt, 5,1 Gew.-% Mangan, 0,3 Gew.-% Natrium und 3,1 Gew.-% Phosphor und wies eine spezifische Oberfläche von 1,6 m²/g auf.

### Beispiele 1 bis 7 (Variation des Lösungsmittels)

Die Beispiele 1 bis 7 wurden als sogenannte Semi-Batch-Versuche in einem 300 ml-Autoklaven aus dem Werkstoff HC4 durchgeführt. Hierzu wurden jeweils außerhalb des Autoklaven 2,46 g eines Raney-Co-Katalysator ("Raney 2724" der Firma W. R. Grace mit 2-6 Gew.-% Al, >86 Gew.-% Co, 0-1 Gew.-% Fe, 1-4 Gew.-% Ni und 1,5-3,5 Gew.-% Cr) mit dem jeweiligen Lösungsmittel aus Tabelle 1 unter N₂-Schutzgas zunächst gewaschen und dann eine Suspension mit der in Tabelle 1 angegebenen Menge an Lösungsmittel hergestellt. Die Suspension wurde dann in den ebenfalls mit N₂ inertisierten Autoklaven überführt. Der Autoklav wurde verschlossen, unter Rühren auf 130°C hochgeheizt und Wasserstoff bis zu einem Gesamtdruck von 8 MPa abs aufgepresst. Innerhalb von 5 Stunden wurde unter weiterem Rühren eine Mischung aus 12,32 g Difluoracetonitril in der in Tabelle 1 angegebenen Menge an Lösungsmittel kontinuierlich bei 130°C zudosiert. Dabei wurde der Gesamtdruck durch Nachpressen von Wasserstoff bei 8 MPa abs konstant gehalten. Nach dem Ende der Zugabe des im Lösungsmittel gelösten Difluoracetonitrils wurde der Autoklav unter weiterem Rühren noch für 1 Stunde bei 130°C und 80 bar Gesamtdruck belassen. Anschließend wurde der Autoklav auf Raumtemperatur abgekühlt, auf Atmosphärendruck entspannt, der enthaltene Katalysator unter N₂-Schutzgas abfiltriert und die filtrierte Reaktionslösung gaschromatographisch analysiert. Der Umsatz von Difluoracetonitril sowie die Ausbeute an 2,2-Difluorethylamin und die daraus errechnete Selektivität zum 2,2-Difluorethylamin sind in Tabelle 1 wiedergegeben.

Sämtliche in den Beispielen 1 bis 7 eingesetzten Lösungsmittel wiesen einen Wassergehalt von 0 Gew.-% bestimmt durch Karl-Fischer-Titration auf.

Die Beispiele zeigen, dass insbesondere in Gegenwart polarer Lösungsmittel Ausbeuten an 2,2-Difluorethylamin von über 80% erzielt werden können.

### Beispiel 8 (Vergleich)

Beispiel 8 wurde wie Beispiel 5 mit Dipropylenglykol-dimethylether als Lösungsmittel durchgeführt, jedoch mit dem Unterschied, dass in den Autoklaven zusätzlich noch 1,03 g LiOH x H₂O (10 mmol/g Katalysator) gelöst in 0,74 g Wasser zugegeben wurden. Das Molverhältnis von Wasser zu Difluoracetonitril betrug 0,41. Bei einem Difluoracetonitril-Umsatz von 99% konnte lediglich eine 2,2-Difluorethylamin-Ausbeute von 31% erhalten werden, was einer 2,2-Difluorethylamin-Selektivität von 32% entspricht.

Das Beispiel zeigt, dass mit 10 mmol/g Katalysator LiOH x H₂O bei einem Molverhältnis von Wasser zu Difluoracetonitril von 0,41 trotz 99%-igem Umsatz nur eine relativ niedrige Ausbeute an 2,2-Difluorethylamin erhalten wurde.

### Beispiele 9 bis 10 (Co-Vollkatalysator)

Die Beispiele 9 bis 10 wurden als sogenannte Semi-Batch-Versuche in einem 300 ml-Autoklaven aus dem Werkstoff HC4 durchgeführt. Hierzu wurden jeweils außerhalb des Autoklaven die in Tabelle 2 angegebene Menge des Katalysators A mit Dipropylenglykol-dimethylether ("Proglyme") als Lösungsmittel unter N₂-Schutzgas zunächst gewaschen und dann eine Suspension mit der in Tabelle 2 angegebenen Menge an Lösungsmittel hergestellt. Die Suspension wurde dann in den ebenfalls mit N₂ inertisierten Autoklaven überführt. Der Autoklav wurde verschlossen, unter Rühren auf 130°C hochgeheizt und Wasserstoff bis zu einem Gesamtdruck von 8 MPa abs aufgepresst. Innerhalb von 5 Stunden wurde unter weiterem Rühren eine Mischung aus der in Tabelle 2 angegebenen Menge Difluoracetonitril in der in Tabelle 2 angegebenen Menge an Dipropylenglykol-dimethylether kontinuierlich bei 130°C zudosiert. Dabei wurde der Gesamtdruck durch Nachpressen von Wasserstoff bei 8 MPa abs konstant gehalten. Nach dem Ende der Zugabe wurde der Autoklav unter weiterem Rühren noch für 1 Stunde bei 130°C und 8 MPa abs Gesamtdruck belassen. Anschließend wurde der Autoklav auf Raumtemperatur abgekühlt, auf Atmosphärendruck entspannt, der enthaltene Katalysator unter N₂-Schutzgas abfiltriert und die filtrierte Reaktionslösung gaschromatographisch analysiert. Der Umsatz von Difluoracetonitril sowie die Ausbeute an 2,2-Difluorethylamin und die daraus errechnete Selektivität zum 2,2-Difluorethylamin sind in Tabelle 2 wiedergegeben.

Die Beispiele zeigen, dass auch durch Einsatz eines sogenannten Vollkatalysators eine relativ hohe Ausbeute an 2,2-Difluorethylamin von über 80% erzielt werden kann.

### Beispiele 11 bis 14 (Variation der Temperatur)

Die Beispiele 11 bis 14 wurden als sogenannte Semi-Batch-Versuche in einem 300 ml-Autoklaven aus dem Werkstoff HC4 durchgeführt. Hierzu wurden jeweils außerhalb des Autoklaven die in Tabelle 3 angegebene Menge eines Raney-Co-Katalysators ("Raney 2724" der Firma W. R. Grace mit 2-6 Gew.-% Al, >86 Gew.-% Co, 0-1 Gew.-% Fe, 1-4 Gew.-% Ni und 1,5-3,5 Gew.-% Cr) mit Dipropylenglykol-dimethylether ("Proglyme") als Lösungsmittel unter N₂-Schutzgas zunächst gewaschen und dann eine Suspension mit der in Tabelle 3 angegebenen Menge an Lösungsmittel hergestellt. Die Suspension wurde dann in den ebenfalls mit N₂ inertisierten Autoklaven überführt. Der Autoklav wurde verschlossen, unter Rühren auf die in Tabelle 3 angegebene Temperatur hochgeheizt und Wasserstoff bis zu einem Gesamtdruck von 8 MPa abs aufgepresst. Innerhalb von 5 Stunden wurde unter weiterem Rühren eine Mischung aus der in Tabelle 3 angegebenen Menge Difluoracetonitril in der in Tabelle 3 angegebenen Menge an Dipropylenglykol-dimethylether kontinuierlich bei der in Tabelle 3 angegebenen Temperatur zudosiert. Dabei wurde der Gesamtdruck durch Nachpressen von Wasserstoff bei 8 MPa abs konstant gehalten. Nach dem Ende der Zugabe wurde der Autoklav unter weiterem Rühren noch für 1 Stunde bei der in Tabelle 3 angegebenen Temperatur und 8 MPa abs Gesamtdruck belassen. Anschließend wurde der Autoklav auf Raumtemperatur abgekühlt, auf Atmosphärendruck entspannt, der enthaltene Katalysator unter N₂-Schutzgas abfiltriert und die filtrierte Reaktionslösung gaschromatographisch analysiert. Der Umsatz von Difluoracetonitril sowie die Ausbeute an 2,2-Difluorethylamin und die daraus errechnete Selektivität zum 2,2-Difluorethylamin sind in Tabelle 3 wiedergegeben.

Die Beispiele zeigen den Einfluss der Temperatur auf die erzielbare Ausbeute an 2,2-Difluorethylamin. Bei 130°C wurde in der vorliegenden Versuchsreihe mit 77% die höchste Ausbeute erreicht.

### Beispiele 15 bis 17 (Einfluss von NH₃)

Die Beispiele 15 bis 17 wurden als sogenannte Semi-Batch-Versuche in einem 300 ml-Autoklaven aus dem Werkstoff HC4 durchgeführt. Hierzu wurden jeweils außerhalb des Autoklaven die in Tabelle 4 angegebene Menge an Raney-Co-Katalysators ("Raney 2724" der Firma W. R. Grace mit 2-6 Gew.-% Al, >86 Gew.-% Co, 0-1 Gew.-% Fe, 1-4 Gew.-% Ni und 1,5-3,5 Gew.-% Cr) mit Dipropylenglykol-dimethylether ("Proglyme") als Lösungsmittel unter N₂-Schutzgas zunächst gewaschen und dann eine Suspension mit der in Tabelle 4 angegebenen Menge an Lösungsmittel hergestellt. Die Suspension wurde dann in den ebenfalls mit N₂ inertisierten Autoklaven überführt. Der Autoklav wurde verschlossen, unter Rühren auf die in Tabelle 4 angegebene Temperatur hochgeheizt und Wasserstoff bis zu einem Gesamtdruck von 8 MPa abs aufgepresst. Innerhalb von 5 Stunden wurde unter weiterem Rühren eine Mischung aus der in Tabelle 4 angegebenen Menge Difluoracetonitril in der in Tabelle 4 angegebenen Menge an Dipropylenglykol-dimethylether kontinuierlich bei der in Tabelle 4 angegebenen Temperatur zudosiert. In diese Menge an Dipropylenglykol-dimethylether wurde vor der Dosierung die in Tabelle 4 angegebene Menge an trockenem NH₃ dosiert. Dabei wurde der Gesamtdruck durch Nachpressen von Wasserstoff bei 8 MPa abs konstant gehalten. Nach dem Ende der Zugabe wurde der Autoklav unter weiterem Rühren noch für 1 Stunde bei der in Tabelle 4 angegebenen Temperatur und 8 MPa abs Gesamtdruck belassen. Anschließend wurde der Autoklav auf Raumtemperatur abgekühlt, auf Atmosphärendruck entspannt, der enthaltene Katalysator unter N₂-Schutzgas abfiltriert und die filtrierte Reaktionslösung gaschromatographisch analysiert. Der Umsatz von Difluoracetonitril sowie die Ausbeute an 2,2-Difluorethylamin und die daraus errechnete Selektivität zum 2,2-Difluorethylamin sind in Tabelle 4 wiedergegeben.

Die Beispiele zeigen den negativen Einfluss von Ammoniak auf die Ausbeute von 2,2-Difluorethylamin. So wurde in Beispiel 15 bei Abwesenheit von NH₃ (Molverhältnis Ammoniak zu Difluoracetonitril gleich 0) eine 2,2-Difluorethylamin-Ausbeute von 77% erzielt. In Beispiel 16 wurde bei einem Molverhältnis Ammoniak zu Difluoracetonitril von 0,051 eine 2,2-Difluorethylamin-Ausbeute von 55% und in Beispiel 17 bei einem Molverhältnis Ammoniak zu Difluoracetonitril von 0,181 eine 2,2-Difluorethylamin-Ausbeute von nur noch 47% erreicht.

**Tabelle 1**

| Bsp. | Lösungsmittel | relative Permittivität εᵣ | Siedepunkt | Lösungsmittel-Menge in Suspension mit Raney-Co Katalysator [g] | Lösungsmittel-Menge in Mischung mit Difluoracetonitril [g] | Umsatz [%] | Ausbeute [%] | Selektivität [%] |
|---|---|---|---|---|---|---|---|---|
| 1 | n-Hexan | 1,88 ^{#1} (25°C) | 68,7°C | 106 | 58 | 100 | 50 | 50 |
| 2 | Toluol | 2,38 ^{#1} (25°C) | 111°C | 139 | 76 | 100 | 55 | 55 |
| 3 | Methyl-tert-butylether | 4,5 ^{#1} (20°C) | 55°C | 118 | 65 | 98 | 74 | 76 |
| 4 | 1,2-Dimethoxylethan | 7,3 ^{#2} (23,6°C) | 85,2°C | 139 | 76 | 99 | 76 | 76 |
| 5 | Dipropylenglykol-dimethylether ("Proglyme") | | 175°C | 145 | 79 | 98 | 79 | 81 |
| 6 | Tetrahydrofuran | 7,58 ^{#1} (25°C) | 66°C | 142 | 78 | 99 | 82 | 83 |
| 7 | N-Methylpyrrolidon | 32,2 ^{#1} (25°C) | 204°C | 105 | 90 | 98 | 82 | 84 |

| | | | | | | | | |
|---|---|---|---|---|---|---|---|---|
| ^{#1} Werte aus C.Reichardt and T.Welton, Solvents and Solvent Effects in Organic Chemistry, 4th edition 2011, Wiley-VCH Verlag ^{#2} Werte aus D.R.Lide and W.M.Haynes, CRC Handbook of Chemistry and Physics, 90th edition 2009, CRC Press | | | | | | | | |

**Tabelle 2**

| Bsp. | Katalysator | Menge Katalysator [g] | Menge Lösungsmittel in Suspension mit Raney-Co Katalysator [g] | Menge Lösungsmittel in Mischung mit Difluoracetonitril [g] | Menge Difluoracetonitril [g] | Umsatz [%] | Ausbeute [%] | Selektivität [%] |
|---|---|---|---|---|---|---|---|---|
| 9 | Katalysator A (gemörsert) | 2,90 | 145 | 79 | 12 | 99 | 68 | 68 |
| 10 | Katalysator A (Katalysatorkorb) | 30 | 120 | 82 | 10 | 99 | 84 | 84 |

**Tabelle 3**

| Bsp. | Temperatur [°C] | Menge Katalysator [g] | Menge Lösungsmittel in Suspension mit Raney-Co Katalysator [g] | Menge Lösungsmittel in Mischung mit Difluoracetonitril [g] | Menge Difluoracetonitril [g] | Umsatz [%] | Ausbeute [%] | Selektivität [%] |
|---|---|---|---|---|---|---|---|---|
| 11 | 70 | 3,20 | 64 | 80 | 16 | 98 | 63 | 64 |
| 12 | 100 | 3,20 | 64 | 80 | 16 | 99 | 63 | 64 |
| 13 | 130 | 3,20 | 64 | 80 | 16 | 99 | 77 | 78 |
| 14 | 160 | 3,20 | 64 | 80 | 16 | 100 | 71 | 71 |

**Tabelle 4**

| Bsp. | Temperatur [°C] | Menge Katalysator [g] | Menge Lösungsmittel in Suspension mit Raney-Co Katalysator [g] | Menge Lösungsmittel in Mischung mit Difluoracetonitril [g] | Menge Difluoracetonitril [g] | Menge NH₃ [g] | Umsatz [%] | Ausbeute [%] | Selektivität [%] |
|---|---|---|---|---|---|---|---|---|---|
| 15 | 130 | 3,20 | 64 | 80 | 16 | 0,00 | 99 | 77 | 78 |
| 16 | 130 | 3,20 | 64 | 80 | 16 | 0,18 | 99 | 55 | 55 |
| 17 | 130 | 3,20 | 64 | 80 | 16 | 0,64 | 99 | 47 | 47 |

## Patentansprüche

1. Verfahren zur Herstellung von 2,2-Difluorethylamin, **dadurch gekennzeichnet, dass** man
a) als Ausgangsstoff Difluoracetonitril einsetzt und dieses
b) mit Wasserstoff
c) bei einem Wasserstoff-Partialdruck von 2 bis 25 MPa und
d) einer Temperatur von 20 bis 250°C
e) in Gegenwart eines heterogenen Katalysators enthaltend mindestens ein Element aus der Gruppe Fe, Co, Ni, Ru, Rh, Pd und Pt hydriert,
wobei man die Hydrierung bei
f) einem Molverhältnis von Wasser zu Difluoracetonitril von 0 bis 0,2; und
g) einem Molverhältnis von Ammoniak zu Difluoracetonitril von 0 bis 1 durchführt.

2. Verfahren nach Anspruch 1, **dadurch gekennzeichnet, dass** man einen heterogenen Katalysator enthaltend Kobalt einsetzt.

3. Verfahren nach Anspruch 1, **dadurch gekennzeichnet, dass** man einen Raney-Katalysator enthaltend Kobalt einsetzt.

4. Verfahren nach den Ansprüchen 1 bis 3, **dadurch gekennzeichnet, dass** man die Hydrierung bei einem Molverhältnis von Wasser zu Difluoracetonitril von 0 bis 0,1 durchführt.

5. Verfahren nach den Ansprüchen 1 bis 4, **dadurch gekennzeichnet, dass** man die Hydrierung bei einem Molverhältnis von Ammoniak zu Difluoracetonitril von 0 bis 0,5 durchführt.

6. Verfahren nach den Ansprüchen 1 bis 5, **dadurch gekennzeichnet, dass** man die Hydrierung bei einem Molverhältnis von organischen und anorganischen Säuren sowie derer Anhydride zu Difluoracetonitril von in Summe 0 bis 0,5 durchführt.

7. Verfahren nach Anspruch 6, **dadurch gekennzeichnet, dass** man die Hydrierung bei einem Molverhältnis von organischen und anorganischen Säuren sowie derer Anhydride zu Difluoracetonitril von in Summe 0 bis 0,25 durchführt.

8. Verfahren nach den Ansprüchen 1 bis 7, **dadurch gekennzeichnet, dass** man die Hydrierung bei einem Molverhältnis von Alkali- und Erdalkali-Hydroxiden und Karbonaten zu Difluoracetonitril von in Summe 0 bis 0,1 durchführt.

9. Verfahren nach Anspruch 8, **dadurch gekennzeichnet, dass** man die Hydrierung bei einem Molverhältnis von Alkali- und Erdalkali-Hydroxiden und Karbonaten zu Difluoracetonitril von in Summe 0 bis 0,05 durchführt.

10. Verfahren nach den Ansprüchen 1 bis 9, **dadurch gekennzeichnet, dass** man die Hydrierung bei einer Temperatur von 50 bis 220°C durchführt.

11. Verfahren nach den Ansprüchen 1 bis 9, **dadurch gekennzeichnet, dass** man die Hydrierung bei einer Temperatur von 120 bis 180°C durchführt.

12. Verfahren nach den Ansprüchen 1 bis 11, **dadurch gekennzeichnet, dass** man die Hydrierung in Gegenwart eines aprotischen Lösungsmittels durchführt, welches bei 25°C eine relative Permittivität εᵣ von ≥ 2 aufweist.

13. Verfahren nach Anspruch 12, **dadurch gekennzeichnet, dass** man aprotische Lösungsmittel einsetzt, die bei einem Druck von 1013,25 hPa abs einen Siedepunkt von ≥ 69°C aufweisen.

14. Verfahren nach den Ansprüchen 12 bis 13, **dadurch gekennzeichnet, dass** man als aprotisches Lösungsmittel Di-n-propylether, Diisopropylether, Di-n-butylether, Diisobutylether, Diisoamylether, Ethylpropylether, Isopropylethylether, Methyl-tert-butylether, Cyclohexylmethylether, 1,2-Dimethoxyethan, Anisol (Methylphenylether), Phenetol (Ethylphenylether), Diphenylether, Dipropylenglykol-dimethylether, 2-(2-Ethoxyethoxy)-2-methylpropan, Diethylenglykol-di-n-butylether, Diethylenglykol-dimethylether, Diethylenglykol-diethylether, Triethylenglykol-dimethylether, Tetraethylenglykol-dimethylether, Methyl-tetrahydrofuran, 1,4-Dioxan, 1,3-Dioxolan, Poly(ethylenglykol)-dimethylether, ethoxylierte Diether eines höhermolekularen Alkohols mit einem Gesamtmolgewicht von über 400 g/mol, Toluol, Xylole, N-Methylpyrrolidon, N-Ethylpyrrolidon, N-Methylpiperidon, N-Ethylpiperidon, N-Methyl-ε-caprolactam und/oder N-Ethyl-ε-caprolactam einsetzt.

15. Verfahren nach den Ansprüchen 12 bis 14, **dadurch gekennzeichnet, dass** man das aprotischen Lösungsmittels in einer Menge von 0,01 bis 100 g pro g Difluoracetonitril einsetzt.

## Claims

1. A process for producing 2,2-difluoroethylamine, wherein
(a) difluoroacetonitrile is employed as starting material and said difluoroacetonitrile
(b) is hydrogenated with hydrogen
(c) at a hydrogen partial pressure of 2 to 25 MPa and
(d) a temperature of 20°C to 250°C
(e) in the presence of a heterogeneous catalyst comprising at least one element from the group Fe, Co, Ni, Ru, Rh, Pd and Pt, wherein the hydrogenation is performed at
(f) a molar ratio of water to difluoroacetonitrile of 0 to 0.2; and
(g) a molar ratio of ammonia to difluoroacetonitrile of 0 to 1.

2. The process according to claim 1, wherein a heterogeneous catalyst comprising cobalt is employed.

3. The process according to claim 1, wherein a Raney catalyst comprising cobalt is employed.

4. The process according to claims 1 to 3 wherein the hydrogenation is performed at a molar ratio of water to difluoroacetonitrile of 0 to 0.1.

5. The process according to claims 1 to 4, wherein the hydrogenation is performed at a molar ratio of ammonia to difluoroacetonitrile of 0 to 0.5.

6. The process according to claims 1 to 5, wherein the hydrogenation is performed at a molar ratio of organic and inorganic acids and anhydrides thereof to difluoroacetonitrile of in total 0 to 0.5.

7. The process according to claim 6, wherein the hydrogenation is performed at a molar ratio of organic and inorganic acids and anhydrides thereof to difluoroacetonitrile of in total 0 to 0.25.

8. The process according to claims 1 to 7, wherein the hydrogenation is performed at a molar ratio of alkali metal and alkaline earth metal hydroxides and carbonates to difluoroacetonitrile of in total 0 to 0.1

9. The process according to claim 8, wherein the hydrogenation is performed at a molar ratio of alkali metal and alkaline earth metal hydroxides and carbonates to difluoroacetonitrile of in total 0 to 0.05.

10. The process according to claims 1 to 9, wherein the hydrogenation is performed at a temperature of 50°C to 220°C.

11. The process according to claim 1 to 9, wherein the hydrogenation is performed at a temperature of 120°C to 180°C.

12. The process according to claims 1 to 11, wherein the hydrogenation is performed in the presence of an aprotic solvent which at 25°C has a relative permittivity εᵣ of ≥ 2.

13. The process according to claim 12, wherein aprotic solvents having a boiling point of 269°C at a pressure of 1013.25 hPa abs are employed.

14. The process according to claims 12 to 13, wherein the aprotic solvent employed is di-n-propyl ether, diisopropyl ether, di-n-butyl ether, diisobutyl ether, diisoamyl ether, ethyl propyl ether, isopropyl ethyl ether, methyl tert-butyl ether, cyclohexyl methyl ether, 1,2-dimethoxyethane, anisole (methyl phenyl ether), phenetol (ethyl phenyl ether), diphenyl ether, dipropylene glycol dimethyl ether, 2-(2-ethoxyethoxy)-2-methylpropane, diethylene glycol din-butyl ether, diethylene glycol dimethyl ether, diethylene glycol diethyl ether, triethylene glycol dimethyl ether, tetraethylene glycol dimethyl ether, methyltetrahydrofuran, 1,4-dioxane, 1,3-dioxolane, poly(ethylene glycol) dimethyl ether, ethoxylated diethers of a relatively high molecular weight alcohol with an overall molecular weight of more than 400 g/mol, toluene, xylenes, N-methylpyrrolidone, N-ethylpyrrolidone, N-methylpiperidone, N-ethylpiperidone, N-methyl-ε-caprolactam and/or N-ethyl-ε-caprolactam.

15. The process according to any of claims 12 to 14, wherein the aprotic solvent is employed in an amount of 0.01 to 100 g per g of difluoroacetonitrile.

## Revendications

1. Procédé de fabrication de 2,2-difluoroéthylamine, **caractérisé en ce que**
a) du difluoroacétonitrile est utilisé en tant que matière première et celui-ci est hydrogéné
b) avec de l'hydrogène
c) à une pression partielle d'hydrogène de 2 à 25 MPa et
d) à une température de 20 à 250 °C,
e) en présence d'un catalyseur hétérogène contenant au moins un élément du groupe constitué par Fe, Co, Ni, Ru, Rh, Pd et Pt,
l'hydrogénation étant réalisée
f) à un rapport molaire entre l'eau et le difluoroacétonitrile de 0 à 0,2 ; et
g) à un rapport molaire entre l'ammoniac et le difluoroacétonitrile de 0 à 1.

2. Procédé selon la revendication 1, **caractérisé en ce qu'**un catalyseur hétérogène contenant du cobalt est utilisé.

3. Procédé selon la revendication 1, **caractérisé en ce qu'**un catalyseur de Raney contenant du cobalt est utilisé.

4. Procédé selon les revendications 1 à 3, **caractérisé en ce que** l'hydrogénation est réalisée à un rapport molaire entre l'eau et le difluoroacétonitrile de 0 à 0,1.

5. Procédé selon les revendications 1 à 4, **caractérisé en ce que** l'hydrogénation est réalisée à un rapport molaire entre l'ammoniac et le difluoroacétonitrile de 0 à 0,5.

6. Procédé selon les revendications 1 à 5, **caractérisé en ce que** l'hydrogénation est réalisée à un rapport molaire entre les acides organiques et inorganiques et leurs anhydrides et le difluoroacétonitrile au total de 0 à 0,5.

7. Procédé selon la revendication 6, **caractérisé en ce que** l'hydrogénation est réalisée à un rapport molaire entre les acides organiques et inorganiques et leurs anhydrides et le difluoroacétonitrile au total de 0 à 0,25.

8. Procédé selon les revendications 1 à 7, **caractérisé en ce que** l'hydrogénation est réalisée à un rapport molaire entre les hydroxydes alcalins et alcalino-terreux et les carbonates et le difluoroacétonitrile au total de 0 à 0,1.

9. Procédé selon la revendication 8, **caractérisé en ce que** l'hydrogénation est réalisée à un rapport molaire entre les hydroxydes alcalins et alcalino-terreux et les carbonates et le difluoroacétonitrile au total de 0 à 0,05.

10. Procédé selon les revendications 1 à 9, **caractérisé en ce que** l'hydrogénation est réalisée à une température de 50 à 220 °C.

11. Procédé selon les revendications 1 à 9, **caractérisé en ce que** l'hydrogénation est réalisée à une température de 120 à 180 °C.

12. Procédé selon les revendications 1 à 11, **caractérisé en ce que** l'hydrogénation est réalisée en présence d'un solvant aprotique qui présente à 25 °C une permittivité relative εᵣ ≥ 2.

13. Procédé selon la revendication 12, **caractérisé en ce que** des solvants aprotiques qui présentent à une pression de 1 013,25 hPa abs un point d'ébullition ≥ 69 °C sont utilisés.

14. Procédé selon les revendications 12 à 13, **caractérisé en ce que** l'éther di-n-propylique, l'éther diisopropylique, l'éther di-n-butylique, l'éther diisobutylique, l'éther diisoamylique, l'éther éthylpropylique, l'éther isopropyléthylique, l'éther méthyl-tert-butylique, l'éther cyclohexyl-méthylique, le 1,2-diméthoxyéthane, l'anisole (éther méthylphénylique), le phénétol (éther éthylphénylique), l'éther diphénylique, l'éther diméthylique de dipropylène glycol, le 2-(2-éthoxyéthoxy)-2-méthylpropane, l'éther di-n-butylique de diéthylène glycol, l'éther diméthylique de diéthylène glycol, l'éther diéthylique de diéthylène glycol, l'éther diméthylique de triéthylène glycol, l'éther diméthylique de tétraéthylène glycol, le méthyl-tétrahydrofurane, le 1,4-dioxane, le 1,3-dioxolane, l'éther diméthylique de poly(éthylène glycol), les diéthers éthoxylés d'un alcool de poids moléculaire élevé ayant un poids moléculaire total de plus de 400 g/mol, le toluène, les xylènes, la N-méthylpyrrolidone, la N-éthylpyrrolidone, la N-méthylpipéridone, la N-éthylpipéridone, le N-méthyl-ε-caprolactame et/ou le N-éthyl-ε-caprolactame sont utilisés en tant que solvant aprotique.

15. Procédé selon les revendications 12 à 14, **caractérisé en ce que** le solvant aprotique est utilisé en une quantité de 0,01 à 100 g par g de difluoroacétonitrile.
